(19) Europäisches Patentamt
European Patent Office
Office européen des brevets

(11) Publication number: 0 344 995
A2

# (12) EUROPEAN PATENT APPLICATION

(21) Application number: 89305386.8

(51) Int. Cl.4: A61K 31/55 , A61K 31/40

(22) Date of filing: 26.05.89

(30) Priority: 01.06.88 US 203173

(43) Date of publication of application:
06.12.89 Bulletin 89/49

(84) Designated Contracting States:
AT BE CH DE ES FR GB GR IT LI LU NL SE

(71) Applicant: E.R. SQUIBB & SONS, INC.
Lawrenceville-Princeton Road
Princeton New Jersey 08543-4000(US)

(72) Inventor: Horovitz, Zola Phillip
30 Philip Drive
Princeton New Jersey(US)

(74) Representative: Thomas, Roger Tamlyn et al
D. Young & Co. 10 Staple Inn
London WC1V 7RD(GB)

**(54) Method for inhibiting loss of cognitive functions employing a calcium channel blocker alone or in combination with an ace inhibitor.**

(57) A method is provided for inhibiting loss of cognitive function, including memory, which may or may not be associated with Alzheimer's disease, in a mammalian species by administering a benzazepine-type calcium channel blocker such as diltiazem, SQ 31,765 or SQ 32,324 alone or in combination with an ACE inhibitor, such as captopril or SQ 29,852, over a prolonged period of treatment.

EP 0 344 995 A2

Xerox Copy Centre

# METHOD FOR INHIBITING LOSS OF COGNITIVE FUNCTIONS EMPLOYING A CALCIUM CHANNEL BLOCKER ALONE OR IN COMBINATION WITH AN ACE INHIBITOR

The present invention relates to a method for inhibiting loss of cognitive functions, including memory, which are associated with different types of dementias in mammalian species by administering a benzazepine-type calcium channel blocker such as diltiazem alone or in combination with an ACE inhibitor, such as captopril, zofenopril or fosinopril over a prolonged period of time.

In accordance with the present invention, a method is provided for inhibiting loss of cognitive functions such as memory, attention span, concentration and ability to learn or for treating or delaying progression of Alzheimer's disease or other types of dementias, in mammalian species over a prolonged period wherein a therapeutically effective amount of a benzazepine-type calcium channel blocker alone or in combination with an angiotensin converting enzyme inhibitor (ACE inhibitor) is systemically, such as orally or parenterally, administered over a prolonged period, to inhibit loss of cognitive function during such period.

Where a combination of a calcium channel blocker and ACE inhibitor are to be used, the calcium channel blocker will be employed in a weight ratio to ACE inhibitor of within the range of from about 0.1:1 to about 10:1 and preferably from about 0.4:1 to about 2.5:1.

The calcium channel blocker also referred to as calcium entry blocker or calcium antagonist which is used herein is preferably diltiazem which is disclosed in U. S. Patent No. 3,562,257 and which has the chemical name 3-(acetyloxy)-5-[2-(dimethylamino)ethyl-2,3-dihydro-2-(4-methoxyphenyl)-1,5-benzothiazepin-4(5H)-one and the structure

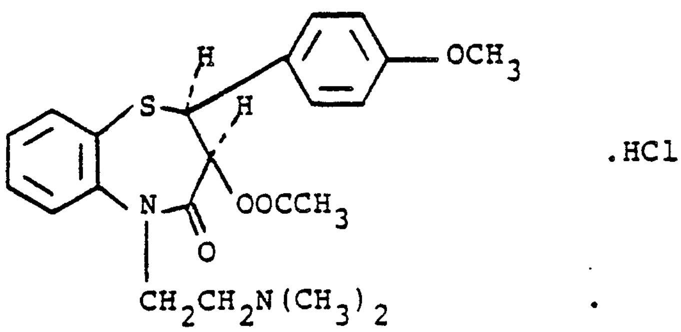

In addition, the calcium channel blocker may be a benzazepine derivative such as disclosed in U. S. patent 4,748,239, and which has the formula

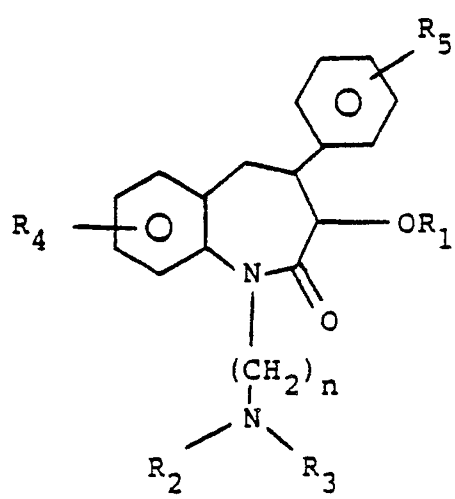

or a pharmaceutically acceptable salt thereof

wherein

$R_1$ is hydrogen, alkyl, alkanoyl, alkenyl, arylcarbonyl, heteroarylcarbonyl or

$-\overset{O}{\overset{\|}{C}}-NX_1X_2$;

$R_2$ and $R_3$ are each independently hydrogen, alkyl, cycloalkyl or arylalkyl or $R_2$ and $R_3$ together with the nitrogen atom to which they are attached are pyrrolidinyl, piperidinyl, or morpholinyl;

$R_4$ and $R_5$ are each independently hydrogen, halogen, alkyl, alkoxy, aryloxy, arylalkoxy, diarylalkoxy, arylalkyl, cyano, hydroxy, alkanoyloxy,

$-O-\overset{O}{\overset{\|}{C}}-NX_1X_2$, fluoro substituted alkoxy, fluoro substituted alkyl, (cycloalkyl)alkoxy, $-NO_2$, $-NX_3X_4$, $-S(O)_m$alkyl, $-S(O)_m$aryl,

$-\overset{O}{\overset{\|}{C}}-X_5$ or $-O-\overset{O}{\overset{\|}{C}}-X_6$;

n is 2 or 3;

m is 0, 1 or 2;

$X_1$ and $X_2$ are each independently hydrogen, alkyl, aryl or heteroaryl, or $X_1$ and $X_2$ together with the nitrogen atom to which they are attached are pyrrolidinyl, piperidinyl or morpholinyl;

$X_3$ and $X_4$ are each independently hydrogen, alkyl, alkanoyl, arylcarbonyl, heteroarylcarbonyl, or

$-\overset{O}{\overset{\|}{C}}-NX_1X_2$;

$X_5$ is hydroxy, alkoxy, aryloxy, amino, alkylamino or dialkylamino; and

$X_6$ is alkyl, alkoxy or aryloxy;

with the proviso that if $R_4$ is a 7-alkyl group, it must have a tertiary carbon atom bonded to the ring;

wherein the term "aryl" refers to phenyl and phenyl substituted with 1, 2 or 3 amino, alkylamino, dialkylamino, nitro, halogen, hydroxyl, trifluoromethyl, alkyl of 1 to 4 carbon atoms, alkoxy of 1 to 4 carbon atoms, alkanoyloxy, carbamoyl, or carboxyl groups; and

the term "heteroaryl" refers to pyridinyl, pyrrolyl, imidazolyl, furyl, thienyl, or thiazolyl.

A preferred such benzazepine derivative has the structure

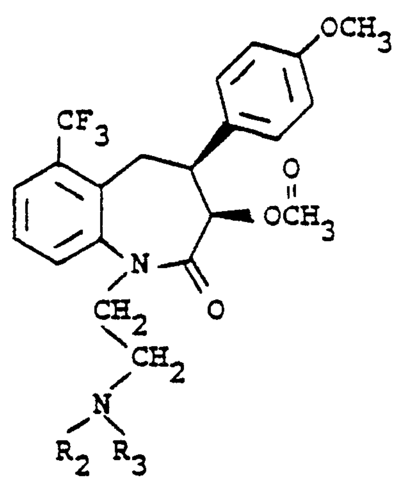

wherein $R_2$ and $R_3$ are each $CH_3$ or one of $R_2$ and $R_3$ is H and the other is $CH_3$, including the hydrochloride salts thereof.

The method of the invention is useful in treating or delaying progression of primary degenerative dementias arising in the senium and presenium such as Alzheimer's disease, Pick's disease and Binswanger's disease, and vascular dementias such as arteriolsclerotic dementias including multiple infarct dementia and Binswanger's disease.

The angiotensin converting enzyme inhibitor which may be employed herein includes substituted proline derivatives, such as any of those disclosed in U. S. Patent No. 4,105,776 to Ondetti et al mentioned above, with captopril, that is, 1-[(2S)-3-mercapto-2-methylpropionyl]-L-proline, being preferred, carboxyalkyl dipeptide derivatives, such as any of those disclosed in U. S. Patent No. 4,374,829, with N-(1-ethoxy carbonyl-3-phenylpropyl)-L-alanyl-L-proline, that is, enalapril, being preferred.

Other examples of angiotensin converting enzyme inhibitors suitable for use herein include any of the phosphonate substituted amino or imino acids or salts disclosed in U. S. Patent No. 4,452,790 with (S)-1-[6-amino-2-[[hydroxy-(4-phenylbutyl)phosphinyl]oxy]-1-oxohexyl]-L-proline (SQ 29,852) being preferred, phosphinylalkanoyl prolines disclosed in U. S. Patent No. 4,168,267 with fosinopril being preferred,

mercaptoacyl derivatives of substituted prolines with zofenopril being preferred, any of the phosphinylalkanoyl substituted prolines disclosed in U. S. Patent No. 4,337,201, and the phosphonamidates disclosed in U. S. Patent No. 4,432,971.

Other examples of ACE inhibitors that may be employed herein include Beecham's BRL 36,378 as disclosed in European patent Nos. 80822 and 60668; Chugai's MC-838 disclosed in CA. 102:72588v and Jap, J. Pharmacol. 40:373 (1986); Ciba-Geigy's CGS 14824 (3-([1-ethoxycarbonyl-3-phenyl-(1S)-propyl]-amino)-2,3,4,5-tetrahydro-2-oxo-1-(3S)-benzazepine-1 acetic acid HCl) disclosed in U.K. Patent No. 2103614 and CGS 16,617 (3(S)-[[(1S)-5-amino-1-carboxypentyl]amino]-2,3,4,5-tetrahydro-2-oxo-1H-1-benzazepine-1-ethanoic acid) disclosed in U. S. Patent No. 4,473,575; cetapril (alacepril, Dainippon) disclosed in Eur. Therap. Res. 39:671 (1986); 40:543 (1986); ramipril (Hoechst) disclosed in Eur. Patent No. 79-022 and Curr. Ther. Res. 40:74 (1986); Ru 44570 (Hoechst) disclosed in Arzneimittelforschung 35:1254 (1985), cilazapril (Hoffman-LaRoche) disclosed in J. Cardiovasc. Pharmacol. 9:39 (1987); $R_o$ 31-2201 (Hoffman-LaRoche) disclosed in FEBS Lett. 165:201 (1984); lisinopril (Merck) disclosed in Curr. Therap. Res. 37:342 (1985) and Eur. patent appl. No. 12-401, indalapril (delapril) disclosed in U. S. Patent No. 4,385,051; rentiapril (fentiapril, Santen) disclosed in Clin. Exp. Pharmacol. Physiol. 10:131 (1983); indolapril (Schering) disclosed in J. Cardiovasc. Pharmacol. 5:643, 655 (1983); spirapril (Schering) disclosed in Acta. Pharmacol. Toxicol. 59 (Supp. 5):173 (1986); perindopril (Servier) disclosed in Eur. J. Clin. Pharmacol. 31:519 (1987); quinapril (warner-Lambert) disclosed in U. S. Patent No. 4,344,949 and CI 925 (Warner-Lambert) ([3S-[2[R(*)R(*)]]3R-(*)]-2-[2-[[1-(ethoxycarbonyl)-3-phenylpropyl]amino[-1-oxopropyl]-1,2,3,4-tetrahydro-6,7-dimethoxy-3-isoquinolinecarboxylic acid HCl) disclosed in Pharmacologist 26:243, 266 (1984), WY-44221 (Wyeth) disclosed in J. Med. Chem. 26:394 (1983).

The disclosure of the above-mentioned patents and other references are incorporated herein by reference.

In carrying out the method of the present invention, calcium channel blocker alone or in combination with the angiotensin converting enzyme inhibitor may be administered to mammalian species, such as monkeys, dogs, cats, rats, humans, etc. and as such may be incorporated in a conventional systemic dosage form, such as a tablet, capsule, elixir or injectable. The above dosage forms will also include the necessary carrier material, excipient, lubricant, buffer, antibacterial, bulking agent (such as mannitol), antioxidants (ascorbic acid of sodium bisulfite) or the like. Oral dosage forms are preferred, although parenteral forms are quite satisfactory as well.

The dose administered must be carefully adjusted according to age, weight and condition of the patient, as well as the route of administration, dosage form and regimen and the desired result.

Thus, for oral administration, a satisfactory result may be obtained employing the calcium channel blocker in an amount within the range of from about 0.01 mg/kg to about 100 mg/kg and preferably from about 0.1 mg/kg to about 25 mg/kg alone or in combination with the ACE inhibitor in an amount within the range of from about 0.01 mg/kg to about 100 mg/kg and preferably from about 0.1 mg/kg to about 25 mg/kg with the calcium channel blocker and ACE inhibitor being employed together in the same oral dosage form or in separate oral dosage forms taken at the same time.

A preferred oral dosage form, such as tablets or capsules, will contain the calcium channel blocker in an amount of from about 0.1 to about 500 mg, preferably from about 125 to about 200 mg, and more preferably from about 25 to about 150 mg, alone or with the ACE inhibitor in an amount of from about 1 to about 350 mg, preferably from about 2 to about 200 mg, and more preferably from about 30 to about 150 mg.

For parenteral administration, the calcium channel blocker will be employed in an amount within the range of from about 0.005 mg/kg to about 10 mg/kg and preferably from about 0.01 mg/kg to about 1 mg/kg, alone or with the ACE inhibitor in an amount within the range of from about 0.005 mg/kg to about 20 mg/kg and preferbly from about 0.01 mg/kg to about 2 mg/kg.

The composition described above may be administered in the dosage forms as described above in single or divided doses of one to four times daily. It may be advisable to start a patient on a low dose combination and work up gradually to a high dose combination.

Tablets of various sizes can be prepared, e.g., of about 50 to 700 mg in total weight, containing one or both of the active substances in the ranges described above, with the remainder being a physiologically acceptable carrier of other materials according to accepted pharmaceutical practice. These tablets can, of course, be scored to provide for fractional doses. Gelatin capsules can be similarly formulated.

Liquid formulations can also be prepared by dissolving or suspending one or the combination of active substances in a conventional liquid vehicle acceptable for pharmaceutical administration so as to provide the desired dosage in one to four teaspoonsful.

Such dosage forms can be administered to the patient on a regimen of one to four doses per day.

According to another modification, in order to more finely regulate the dosage schedule, the active substances may be administered separately in individual dosage units at the same time or carefully coordinated times. Since blood levels are built up and maintained by a regulated schedule of administration, the same result is achieved by the simultaneous presence of the two substances. The respective substances can be individually formulated in separate unit dosage forms in a manner similar to that described above.

Fixed combinations of calcium channel blocker and ACE inhibitor are more convenient and are preferred, especially in tablet or capsule form for oral administration.

In formulating the compositions, the active substances, in the amounts described above, are compounded according to accepted pharmaceutical practice with a physiologically acceptable vehicle, carrier, excipient, binder, preservative, stabilizer, flavor, etc., in the particular type of unit dosage form.

Illustrative of the adjuvants which may be incorporated in tablets are the following: a binder such as gum tragacanth, acacia, corn starch or gelatin; an excipient such as dicalcium phosphate or cellulose; a disintegrating agent such as corn starch, potato starch, alginic acid or the like; a lubricant such as stearic acid or magnesium stearate; a sweetening agent such as sucrose, lactose or saccharin; a flavoring agent such as orange, peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets or capsules may be coated with shellac, sugar or both. A syrup of elixir may contain the active compound, water, alcohol or the like as the carrier, glycerol as solubilizer, sucrose as sweetening agent, methyl and propyl parabens as preservatives, a dye and a flavoring such as cherry or orange.

Many of the active substances described above form commonly known, pharmaceutically acceptable salts such as alkali metal and other common basic salts or acid addition salts, etc. References to the base substances are therefore intended to include those common salts known to be substantially equivalent to the parent compound.

The formulations as described above will be administered for a prolonged period, that is, for as long as the potential for onset of loss of cognitive function remains or the symptoms continue. Sustained release forms of such formulations which may provide such amounts biweekly, weekly, monthly and the like may also be employed. A dosing period of at least one to two weeks are required to achieve minimal benefit.

The following Examples represent preferred embodiments of the present invention.

## Example 1

A diltiazem formulation suitable for oral administration in inhibiting loss of cognitive functions is set out below.

1000 tablets each containing 100 mg of diltiazem were produced from the following ingredients.

| | |
|---|---|
| Diltiazem | 100 g |
| Corn starch | 50 g |
| Gelatin | 7.5 g |
| Avicel (microcrystalline cellulose) | 25 g |
| Magnesium stearate | 2.5 g |

The diltiazem and corn starch are admixed with an aqueous solution of the gelatin. The mixture is dried and ground to a fine powder. The Avicel and then the magnesium stearate are admixed with the granulation. This is then compressed in a tablet to form 1000 tablets each containing 100 mg of active ingredient which is used for inhibiting loss of cognitive functions.

## Example 2

1000 tablets each containing 200 mg of SQ 32,324 are produced from the following ingredients:

| | |
|---|---|
| (d-cis)-3-(acetyloxy)-1,3,4,5-tetrahydro-4-(4-methoxyphenyl)-1-[2-(methylamino)ethyl]-6-(trifluoromethyl)-2H-1-benzazepin-2-one, monohydrochloride salt (SQ 32,324) | 200 g |
| Lactose | 100 g |
| Avicel | 150 g |
| Corn starch | 50 g |
| Magnesium stearate | 5 g |

SQ 32,324 lactose and Avicel are admixed, then blended with the corn starch. Magnesium stearate is added. The dry mixture is compressed in a tablet press to form 1000 505 mg tablets each containing 200 mg of active ingredient. The tablets are coated with a solution of Methocel E 15 (methyl cellulose) including as a color a lake containing yellow #6. The resulting tablets are useful in inhibiting loss of cognitive functions.

## Example 3

Two piece #1 gelatin capsules each containing 250 mg of diltiazem are filled with a mixture of the following ingredients:

| | |
|---|---|
| Diltiazem | 250 mg |
| Magnesium stearate | 7 mg |
| USP lactose | 193 mg. |

The resulting capsules are useful in inhibiting loss of cognitive functions.

## Example 4

An injectable solution for use in inhibiting loss of cognitive functions is produced as follows:

| | |
|---|---|
| (d-cis)-3-(acetyloxy)-1-[2-dimethylamino)ethyl]-1,3,4,5-tetrahydro-4-(4-methoxyphenyl)-6-(trifluoromethyl)-2H-1-benzazepin-2-one, monohydrochloride (SQ 31,765) | 500 mg |
| Methyl paraben | 5 mg |
| Propyl paraben | 1 mg |
| Sodium chloride | 25 g |
| Water for injection qs. | 5 l. |

SQ 31,765, preservatives and sodium chloride are dissolved in 3 liters of water for injection and then the volume is brought up to 5 liters. The solution is filtered through a sterile filter and aseptically filled into presterilized vials which are then closed with presterilized rubber closures. Each vial contains 5 ml of solution in a concentration of 100 mg of active ingredient per ml of solution for injection.

## Example 5

A captopril-diltiazem formulation suitable for oral administration in inhibiting loss of cognitive function is set out below.

1000 tablets each containing 100 mg of 1-[(2S)-3-mercapto-2-methylpropionyl]-L-proline and 100 mg of diltiazem are produced from the following ingredients:

| | |
|---|---|
| 1-(2S)-3-mercapto-2-methylpropionyl]-L-proline (captopril) | 100 g |
| Diltiazem | 100 g |
| Corn starch | 50 g |
| Gelatin | 7.5 g |
| Avicel (microcrystalline cellulose) | 25 g |
| Magnesium stearate | 2.5 g |

The captopril, diltiazem and corn starch are admixed with an aqueous solution of the gelatin. The mixture is dried and ground to a fine powder. The Avicel and then the magnesium stearate are admixed with the granulation. This is then compressed in a tablet to form 1000 tablets each containing 200 mg of active ingredients which is used for inhibiting loss of cognitive function.

## Example 6

By substituting 100 g of 1-(3-mercapto-2-D-methylpropanoyl)-L-proline for the captopril in Example 5, 1000 tablets each containing 100 mg of the 1-(3-mercapto-2-D-methylpropanoyl)-L-proline and 100 mg diltiazem are produced which is useful in inhibiting loss of cognitive function.

## Example 7

1000 tablets each containing 200 mg of SQ 29,852 and 200 mg SQ 32,324 are produced from the following ingredients:

| | |
|---|---|
| SQ 29,852 | 200 g |
| SQ 32,324 | 200 g |
| Lactose | 100 g |
| Avicel | 150 g |
| Corn starch | 50 g |
| Magnesium stearate | 5 g |

The SQ 29,852, SQ 32,324, lactose and Avicel are admixed, then blended with the corn starch. Magnesium stearate is added. The dry mixture is compressed in a tablet press to form 1000 505 mg tablets each containing 200 mg of each active ingredient. The tablets are coated with a solution of Methocel E 15 (methyl cellulose) including as a color a lake containing yellow #6. The resulting tablets are useful in inhibiting loss of cognitive function.

## Example 8

An injectable solution for use in treating or preventing loss of cognitive function is produced as follows:

| | |
|---|---|
| SQ 29,852 | 500 mg |
| Diltiazem | 300 mg |
| Methyl paraben | 5 g |
| Propyl paraben | 1 g |
| Sodium chloride | 25 g |
| Water for injection qs. | 5 L. |

The SQ 29,852, diltiazem, preservatives and sodium chloride are dissolved in 3 liters of water for injection and then the volume is brought up to 5 liters. The solution is filtered through a sterile filter and aseptically filled into presterilized vials which are then closed with presterilized rubber closures. Each vial contains 5 ml of solution in a concentration of 100 mg of active ingredient per ml of solution for injection.

## Example 9

Tablets for use in inhibiting loss of cognitive function are prepared as described in Example 5 except that N-(1-ethoxycarbonyl-3-phenylpropyl)-L-alanyl-L-proline (enalapril) is used in place of captopril and SQ 32,324 is used in place of diltiazem.

## Example 10

Tablets for use in inhibiting loss of cognitive function are prepared following the procedure of Example 5 except that zofenopril is employed in place of captopril.

## Example 11

Tablets for use in inhibiting loss of cognitive function are prepared following the procedure of Example 5 except that fosinopril is employed in place of captopril.

## Example 12

Tablets for use in inhibiting loss of cognitive function are prepared following the procedure of Example 5 except that SQ 29,852 is employed in place of captopril.

## Example 13

The following experiments were carried out to demonstrate the effectiveness of benzazepine-type calcium channel blockers alone or in combination with ACE inhibitors in inhibiting loss of cognitive functions.

Adult male Sprauge-Dawley rats (Charles River, Wilmington, MA), age 25 weeks and weighing 350-400 g, are separately housed in stainless steel cages with continuous access to food and water, a 12-hour light-dark cycle, and constant room temperature of 22° to 24° C. All testing took place approximately 6 hours into the dark component of the light-dark cycle and was conducted in a dimly lighted soundproof room using a standard shuttle box device (Lehigh Valley Electronics #146-04), a plexiglass chamber (50 x 20 x 20

cm) divided by a center barrier 7 cm in height. The conditioned stimulus consisted of a 10-second tone provided by a Sonlert mounted in the midpoint of the ceiling of the chamber. Floor current of 0.8 mA was delivered by a constant current shocker-scrambler (Lehigh Valley Electronics #133-33).

The day before the initiation of training each animal was allowed to explore the experimental chamber for 10 minutes without any tone or shock. Training was conducted for 15 days following the day of experimental chamber exploration. Each animal received 20 trials per day on a 30-second variable interval schedule. No drug treatment was administered during the training period. Shock could be avoided by shuttling from one side of the center barrier to the other during the 10-second tone period. If an animal did not cross the center barrier during this period, the tone remained on and the floor shock was delivered until the animal escaped to the other half of the chamber. Animals which consistently remained on the center barrier were removed from the study. Automatic counters recorded the number of avoidance responses, escapes, and intertrial crossings, while a running time meter recorded the total shock duration for each animal.

Animals not meeting the admittance criterion of correct avoidance rsponding on at least 85% of the trials for 4 out of the last 5 days of training were removed from the study. A total of 99 rats reaching the admittance criterion were tested for extinction of conditioned avoidance response (CAE) during 14 days. They were randomly assigned to captopril (ACE inhibitor) (10 mg/kg and 30 mg/kg), zofenopril (ACE inhibitor) (10 mg/kg and 30 mg/kg), fosinopril (ACE inhibitor) (10 mg/kg and 30 mg/kg), methyldopa (no ACE inhibiting acitivity) (10 mg/kg and 30 mg/kg), epicaptopril (a derivative of captopril which does not have ACE inhibiting activity) (10 mg/kg and 30 mg/kg), diltiazem (10 mg/kg and 30 mg/kg) and saline control with each test group at each dosage comprising 9 rats. All solutions were prepared fresh and administered i.p. (1 mg/ml volume) on the 2 days prior to testing and then 1 hour before it. Testing consisted of 20 trials per day identical to those previously described, except that no shock was administered if an animal failed to shuttle during the 10-second tone period. The tone was simply discontinued and the testing proceeded.

Two-way analysis of variance of the CAE data yielded an overall significant difference in the rate of shuttle extinction between treatment groups.

The findings indicate that diltiazem and ACE inhibitors, namely, fosinopril, zofenopril and captopril possess protective effects on memory of previously learned tasks while compounds such as epicaptopril and methyldopa which do not have ACE inhibiting activity do not have protective effects against loss of memory of previously learned tasks.

It will also be appreciated that all of the above compounds and formulations may be employed in treating or delaying progression of Alzheimer's disease.

In addition, the calcium channel blocker may be a benzazepine derivative such as disclosed. in U. S. patent application Serial No. 42,187, filed April 24, 1987, such as SQ 32,324 and SQ 31,765.

## Example 14

The following experiments were carried out to demonstrate the effectiveness of diltiazem to improve cognition and cognitive impairment.

### ABILITY TO IMPROVE BASIC PERFORMANCE AND TO ANTAGONIZE A SCOPOLAMINE IMPAIRMENT IN A MOUSE HABITUATION TEST

#### Methods

The studies used a black:white test box procedure as described below. Male albino (BKW) mice were used, initially weighing 25-30 g. In their home cage, mice were housed in groups of 10 and given free access to food and water. The mice were kept on a 12 hour light and 12 hour dark cycle with lights off at 8:00 a.m. and on at 8:00 p.m.

The test box consisted of an open-topped box (45 x 27 x 27 cm), 40% of the area painted black and illuminated with a dim red light (1 x 60 W), the other painted white and brightly illuminated with white light (1 x 60W) located 17 cm above the box. Access between the two areas was enabled by means of a 7.5 x 7.5 cm opening located at floor level in the center of the partition (which also served to prevent diffusion of

light between the two compartments of the test box). The floor area was lined into 9 cm squares.

The habituation test was carried out daily by placing mice in the center of the white section of the test box (mice taken from dark home environment in a dark container, to the experimental room maintained in low red lighting, and would normally be averse to the bright white conditions). Testing was carried out between 8:30 and 12:30 p.m. The test period was 5 minutes per day. Behavior was assessed via remote video recording, and the following measures taken:

1. Latency to move from the white to the black section (sec).
2. Numbers of exploratory rears in the white and black sections during the 5 minute test.
3. Numbers of line crossings (exploratory locomotion) in the white and black sections during the 5 minute test.
4. Time spent in the black section of the box during the 5 minute test.
5. Numbers of transitions between the black and white sections of the test box during the 5 minute test (since this parameter was not changed in any situation in the present studies, data for transitions is not given or commented on further).

Generally, as animals habituated to the test system, they would move into the black section of the box where behavioral exploration was exhibited as exploratory rears and line crossings.

Scopolamine was used at a dose of 0.25 mg/kg i.p. to disrupt habituation patterns. This could be achieved by a single acute challenge with scopolamine which disrupted the learning patterns on the day of treatment, with subsequent recovery, or by continued daily treatment with scopolamine 1 hour before test. The dose of scopolamine was carefully selected as one which did not cause autonomic disturbance (0.25 mg/kg i.p. methyl scopolamine failed to influence behavior). Under the influence of 0.25 mg/kg i.p. scopolamine mice would go to the door in the partition, investigate the opening and pass the head or body through, but without association of the dark environment with escape from the brightly-lit averse environment.

## Results

The normal learning curve for mice in the habituation test was 5-6 days as evidenced by reduced rearings and line crossings in the white compartment, increased in the black, reduced latency to move to the black and increased % of time spent in the black. Acutely administered scopolamine causes impairment in control animals. Example data is given here for rears: mice had 'learned' to avoid the white averse environment and by day 6 were carrying out most of their behavior in the black - this was prevented by scopolamine which caused an impairment characterized by increased activity in the white, decreased in the black. This impairment caused by scopolamine can be prevented by arecoline. The selection of dose and route for arecoline are critical to avoid unwanted autonomic disturbance. The arecoline is given continuously by intraperitoneal infusion from Alzet osmotic minipumps at a dose of 50 mg/kg/day. It is interesting that while the continuous treatment with this dose of arecoline inhibited the scopolamine impairment of habituation, the time course of the basic 'learning' or habituation was unaffected by the presence of arecoline. This contrasts with findings for the diltiazem.

Using the same procedure as described above, control mice and mice treated with diltiazem were subject to the habituation procedure and challenged with scopolamine on days 6 and 10. Firstly, it was seen that the basal learning procedure was speeded by treatment with diltiazem. Secondly, the treatments with diltiazem were shown to completely antagonize the impairments caused by scopolamine.

Assessments of the potential of hydergine to improve cognitive function in the mouse habituation test utilized the same test protocol as described so far for arecoline and diltiazem Hydergine was obtained as a proprietary product and the human dose titrated to mouse for single daily challenge, orally, 60 minutes before test. Treatment with hydergine was clearly shown to enhance 'learning' in the mouse habituation test. Rearing in the white section rapidly diminished as this behavior correspondingly increased in the black, and crossings in the white decreased significantly below control values by day 2 of testing, again with corresponding increases in the black and increased % of time in the black was significant on day 2 as were the reductions in latencies to move from the white to the black section on days 2, 3 and 4 of testing.

The treatment with hydergine was not associated with any anxiolytic potential and the dose regime was maintained constant at 0.1mg/kg p.o. daily. After 4 days some motor impairment and sedation developed in a small proportion of animals; this particularly influenced the latency to move from the white environment and data for such animals had to be excluded from analyses.

A very important observation was that while hydergine (like diltiazem but in contrast to arecoline) could

enhance basal learning, it was not able to antagonize the influence of scopolamine to impair performance whether measured as changed rearing, changed line crossings or changed % time in black, and latency to move out of the white, aversive environment. This failure to antagonize, indeed, to any way influence the impairment caused by scopolamine contrasts with the marked antagonistic effects of arecoline and diltiazem.

In a further series of experiments mice were allowed to habituate for 10 days and then were challenged daily with scopolamine, 0.25mg/kg. The habituation was impaired throughout the time of scopolamine challenge. If, after impairment with scopolamine was established, mice were given arecoline (50 mg/kg/day by intraperitoneal infusion from Alzet osmotic minipumps), or diltiazem daily with the scopolamine treatment, then the scopolamine impairment was completely prevented.

## CONCLUSIONS

## ASSESSMENT OF ABILITY TO IMPROVE COGNITION AND COGNITIVE IMPAIRMENT

A mouse habituation test was used in which mice were repeatedly placed in the white compartment of a white:black test box. On repeated exposure to the test situation, mice 'learn' to avoid the averse white, brightly-lit environment and move rapidly into the black where they spend a larger proportion of time and show most exploratory rearings and line crossings.

The habituation (learning) time is 5-6 days. This basic 'learning' time was not influenced by arecoline (50 mg/kg/day given by continuous intraperitoneal infusion: dose and route selected to avoid unwanted autonomic effects). However, this dose of arecoline successfully antagonised an impairment in habituation caused by acute challenge with scopolamine (0.25 mg/kg i.p., dose again carefully selected to avoid excessive peripheral autonomic disturbance, and particularly to avoid influence on vision which can influence performance in the test: lack of effect on vision was established by visual observation and by measurement of pupil function). Methyl scopolamine at a dose of 0.25 mg/kg i.p. failed to influence mouse habituation. The effect of scopolamine was marked: animals which had learned to avoid the white environment failed to enter the black, excepting for short periods of time, even though they easily found the door, and thus the rapid exit into the black and avoidance of the white environment was prevented by scopolamine treatment.

In contrast to arecoline, the speed of habituation was enhanced by treatment with low doses of diltiazem. Diltiazem was found to antagonize the impairment in habituation performance caused by acute challenge with scopolamine.

Hydergine (0.1 mg/kg p.o. once daily) was shown to speed the habituation process in a similar manner to diltiazem (both in contrast to the failure of arecoline), but hydergine treatment failed to influence the impairment in habituation caused by acute challenge with scopolamine (which contrasts with the actions of both diltiazem and the cholinomimetic agent).

Further studies allowed habituation to progress for 10 days before continuous impairment by scopolamine given daily for up to 14 days. This persistent impairment caused by scopolamine could be antagonized by arecoline (50 mg/kg/day by intraperitoneal infusion) and by diltiazem.

## Claims

1. Use of a benzazepine-type calcium channel blocker for the manufacture of a medicament for inhibiting loss of cognitive function over a prolonged period of time in a mammalian specie to which or to whom an angiotensin converting enzyme inhibitor is optionally also being administered.

2. The use as defined in Claim 1 wherein the calcium channel blocker is diltiazem.

3. The use as defined in Claim 1 wherein the calcium channel blocker has the formula

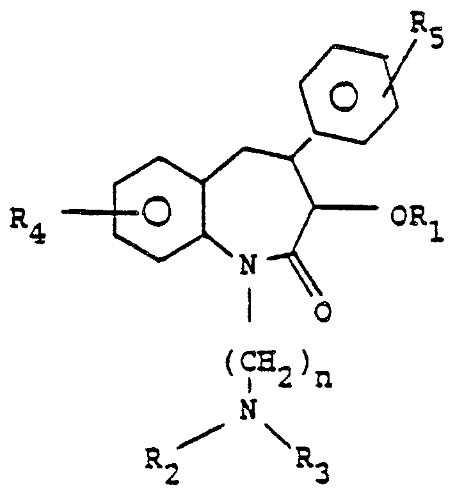

or a pharmaceutically acceptable salt thereof
wherein
$R_1$ is hydrogen, alkyl, alkanoyl, alkenyl, arylcarbonyl, heteroarylcarbonyl or
$-\overset{O}{\overset{\|}{C}}-NX_1X_2$;
$R_2$ and $R_3$ are each independently hydrogen, alkyl, cycloalkyl or arylalkyl or $R_2$ and $R_3$ together with the nitrogen atom to which they are attached are pyrrolidinyl, piperidinyl, or morpholinyl;
$R_4$ and $R_5$ are each independently hydrogen, halogen, alkyl, alkoxy, aryloxy, arylalkoxy, diarylalkoxy, arylalkyl, cyano, hydroxy, alkanoyloxy,
$-O-\overset{O}{\overset{\|}{C}}-NX_1X_2$, fluoro substituted alkoxy, fluoro substituted alkyl, (cycloalkyl)alkoxy, $-NO_2$, $NX_3X_4$, $-S(O)_m$alkyl, $-S(O)_m$aryl,
$-\overset{O}{\overset{\|}{C}}-X_5$ or $-O-\overset{O}{\overset{\|}{C}}-X_6$;
n is 2 or 3;
m is 0, 1 or 2;
$X_1$ and $X_2$ are each independently hydrogen, alkyl, aryl or heteroaryl, or $X_1$ and $X_2$ together with the nitrogen atom to which they are attached are pyrrolidinyl, piperidinyl or morpholinyl;
$X_3$ and $X_4$ are each independently hydrogen, alkyl, alkanoyl, arylcarbonyl, heteroarylcarbonyl, or
$-\overset{O}{\overset{\|}{C}}-NX_1X_2$;
$X_5$ is hydroxy, alkoxy, aryloxy, amino, alkylamino or dialkylamino; and
$X_6$ is alkyl, alkoxy or aryloxy;
with the proviso that if $R_4$ is a 7-alkyl group, it must have a tertiary carbon atom bonded to the ring;
wherein the term "aryl" refers to phenyl and phenyl substituted with 1, 2 or 3 amino, alkylamino, dialkylamino, nitro, halogen, hydroxyl, trifluoromethyl, alkyl of 1 to 4 carbon atoms, alkoxy of 1 to 4 carbon atoms, alkanoyloxy, carbamoyl, or carboxyl groups;
the term "heteroaryl" refers to pyridinyl, pyrrolyl, imidazolyl, furyl, thienyl, or thiazolyl.

4. The use as defined in Claim 3 wherein the calcium channel blocker is (d-cis)-3-(acetyloxy)-1-[2-dimethylamino)ethyl]-1,3,4,5-tetrahydro-4-(4-methoxyphenyl)-6-(trifluoromethyl)-2H-1-benzazepin-2-one, monohydrochloride (SQ 31,765) or (d-cis)-3-(acetyloxy)-1,3,4,5-tetrahydro-4-(4-methoxyphenyl)-1-[2-(methylamino)ethyl]-6-(trifluoromethyl)-2H-1-benzazepin-2-one, monohydrochloride salt (SQ 32,324).

5. The use as defined in any preceding claim wherein the angiotensin converting enzyme inhibitior is a phosphonate substituted amino or imino acid or salt thereof,, a substituted proline derivative, a carboxyalkyl dipeptide derivative, a phosphinylalkanoyl proline derivative or a phosphonamidate derivative.

6. Ihe use as defined in any one of Claims 1-4 wherein said angiotensin converting enzyme inhibitor is a substituted proline derivative.

7. The use as defined in any one of Claims 1-4 wherein said angiotensin converting enzyme inhibitor is a carboxyalkyl dipeptide derivative.

8. The use es defined in any one of Claims 1-4 wherein said angiotensin converting enzyme inhibitor is a phosphinylalkanoyl proline derivative.

9. The use as defined in any one of Claims 1-4 wherein said angiotensin converting enzyme inhibitor is a phosphonamidate derivative.

10. The use as defined in any one of Claims 1-4 wherein said angiotensin converting enzyme inhibitor is a phosphonate substituted amino or imino acid or salt thereof.

11. The use as defined in any one of Claims 1-4 wherein said angiotensin converting enzyme inhibitor is captopril, enalapril, 1-[N-[hydroxy-(4-phenylbutyl)phosphinyl]-2-alanyl]-L-proline or its disodium salt, SQ 29,852, zofenopril, fosinopril or (S)-1-[6-amino-2-[[hydroxy(4-phenylbutyl)phosphinyl]oxy]-1-oxohexyl]-L-proline.

12. The use as defined in Claim 1 wherein said calcium channel blocker is diltiazem, (d-cis)-3-(acetyloxy)-1-[2-dimethylamino)ethyl]-1,3,4,5-tetrahydro-4-(4-methoxyphenyl)-6-(trifluoromethyl)-2H-1-benzazepin-2-one, monohydrochloride (SQ 31,765) or (d-cis)-3-(acetyloxy)-1,3,4,5-tetrahydro-4-(4-methoxyphenyl)-1-[2-(methylamino)ethyl]-6-(trifluoromethyl)-2H-1-benzazepin-2-one, monohydrochloride salt (SQ 32,324) and the medicament is for administraticn systemically in an amount of from about 0.1 to about 500 mg/1 to 4 times a day and when used said angiotensin converting enzyme inhibitor is captopril and is administered systemically in an amount of from about 0.1 to about 500 mg/1 or 4 times a day.

13. Use of a benzazepine-type calcium channel blocker for the manufacture of a medicament for treating or delaying progression of Alzheimer's disease in a mammalian specie to which or to whom an angiotensin converting enzyme inhibitor is optionally also being administered.

14. The use as defined in Claim 13 wherein the calcium channel blocker is diltiazem, (d-cis)-3-(acetyloxy)-1-[2-dimethylamino)ethyl]-1,3,4,5-tetrahydro-4-(4-methoxyphenyl)-6-(trifluoromethyl)-2H-1-benzazepin-2-one, monohydrochloride (SQ 31,765) or (d-cis)-3-(acetyloxy)-1,3,4,5-tetrahydro-4-(4-methoxyphenyl)-1-[2-(methylamino)ethyl]-6-(trifluoromethyl)-2H-1-benzazepin-2-one, monohydrochloride salt (SQ 32,324) and when used the angiotensin converting enzyme inhibitor is captopril, SQ 29,852, zofenopril, fosinopril, enalapril or (S)-1-[6-amino-2-[[hydroxy(4-phenylbutyl)phosphinyl]oxy]-1-oxohexyl-L-proline.

15. The use as defined in any preceding claim wherein the angiotensin converting enzyme inhibitor is also used for the manufacture of the medicament.